# EUROPEAN PATENT APPLICATION

(11) **EP 1 465 099 A1**
(43) Date of publication of application: **06.10.2004**
(21) Application number: 04251604.7
(22) Date of filing: 19.03.2004
(51) Int. Cl.: G06F 17/60, G06F 19/00

(54) **Medical image photographing system with a portable terminal**

(30) Priority: 28.03.2003 JP 2003091535; 28.03.2003 JP 2003092407
(71) Applicant: KONICA MINOLTA HOLDINGS, INC., Tokyo 100-0005 (JP)
(72) Inventor: Shiibasi, Takao, Hachioji-shi, Tokyo 192-8505 (JP); Moriyama, Naoto, Hachioji-shi, Tokyo 192-8505 (JP); Motoki, Wataru, Hachioji-shi, Tokyo 192-8505 (JP)
(74) Representative: Rees, Alexander Ellison

(57) **Abstract**

A medical image photographing system includes: an information management apparatus for readably holding photographing order information including a patient ID and radiographing conditions, and for sending the photographing order information; a control apparatus for obtaining the photographing order information from the information management apparatus through a communication network; and a portable terminal for displaying the photographing order information obtained from the control apparatus, obtaining the information out of the displayed photographing order information, obtaining a panel identification information, and storing into a memory the obtained panel identification information, wherein the portable terminal has an input unit for inputting various commands.

## Description

### BACKGROUND OF THE INVENTION

### Field of the Invention

The present invention relates to a medical image photographing system that obtains a medical image such as a radiograph.

### Description of the Related Art

There has been recently developed a medical image photographing system having a radiographing apparatus such as a CR (Computed Radiography) apparatus which obtains radiographs widely used as medical images as digital image data.

The above-described radiographing apparatus uses, for example, a radiograph converting panel (hereinafter referred to as "imaging plate") in which a photostimulable phosphor layer is formed. This imaging plate accumulates the radiation energy corresponding to the quantity of radiation transmitted through each part of a subject, and emits the accumulated radiation energy as fluorescence with irradiation of excitation light, such as infrared rays. The light signal due to this fluorescence is photoelectrically converted into radiograph signals by a photoelectric transducer, such as a semiconductor device or the like. The radiograph thus obtained is, after image processing, visualized through a film or a displaying device, or stored into a database with patient information to be utilized for various medical actions.

A radiography operator carries out X-ray radiography using the radiographing apparatus installed in a radiographing room within a hospital, based on photographing order information issued from HIS (Hospital Information System) or RIS (Radiology Information System) which manages examination information within hospital facilities or a radiology department, respectively.

The photographing order information has, for example, the name of a patient, patient ID (identification) information, sex, age, hospital room (having a ward and a floor), patient location (the location of a bed), requesting department, requesting doctor's name, radiographic part, the size and the sheet number of radiographing panels, operator ID of the radiography operator in charge, a flag showing complete/incomplete photography, etc.

Recently, there has been realized a mobile radiographing apparatus (hereinafter "portable radiographing apparatus") capable of X-ray radiographing at a bedside in a hospital room accommodating a patient (see, for example, Japanese Patent Application Publication (Unexamined) tokukai-2000-139888 and tokukai-2002-125960). Utilization of this portable radiographing apparatus allows a patient, who has difficulty in walking (movement) due to a serious illness and can not move to the radiographing room, to be easily photographed by X-rays. Further, if a medical image reading apparatus for reading a radiograph from the imaging plate is installed at a predetermined location or a nurses' station on each floor of the ward, the radiography operator can easily confirm the photographed result without moving floor to floor.

The above-described imaging plate is housed in a specialized case called a cassette, and each cassette and/or imaging plate has its own particular identification number, which allows the cassette and/or imaging plate to be identified.

The earlier development described above, however, has a problem. Since equipments, such as the portable radiographing apparatus and the like, require complicated operations, an unexpected accident could occur, if a radiography operator holds both a radiographing panel and a portable terminal, that is, if the radiography operator operates the portable terminal with the panel held in his one-side arm and the portable terminal held in the other hand when quick operations are required when the photography is carried out at a bedside.

Although the prompt and reliable preparation for the radiography is necessary at the bedside of a hospital room, it is a fact that the labor hour and the procedure took in the preparation for the radiography are a little different depending on a radiography operator. Therefore, the time took in the preparation for the radiography is sometimes lopsided and long on average, or there is the possibility that a wrong operation is induced by such different preparation for the radiography.

### SUMMARY OF THE INVENTION

An object of the present invention is to provide a medical image photographing system capable of making easier medical photographing operation carried out by a radiography operator at the bedside of a patient and avoiding an unexpected accident due to a wrong operation or the like.

In order to solve the above-described problems, in accordance with the first aspect of the present invention, a medical image photographing system comprises:
an information management apparatus for readably holding photographing order information having a patient ID particular to a patient to be photographed medical images and radiographing conditions having radiographic part, and for sending the photographing order information outside according to an inputted command;
a control apparatus for obtaining the photographing order information from the information management apparatus through a communication network built in a hospital; and
a portable terminal for displaying the photographing order information obtained from the control apparatus on a screen, obtaining the information relating to the particular patient out of the displayed photographing order information, obtaining a panel identification information particular to a radiographing panel to be used for photographing a medical image of the patient, and storing into a memory the obtained panel identification information made correspondence with the photographing order information,
wherein the portable terminal has input keys for inputting various commands, and inputs, with input operations by the input keys, a command to display on the screen the photographing order information obtained from the control apparatus, a command to obtain the information relating to the particular patient out of the displayed photographing order information, a command to obtain the panel identification information particular to the radiographing panel to be used for photographing the medical image of the patient, and a command to store into the memory the obtained panel identification information made correspondence with the photographing order information.

According to this invention, since a radiography operator can easily operate the portable terminal with one hand only, the radiography operator can operate the portable terminal quickly and securely even if the radiography operator holds both the radiographing panel and the portable terminal, that is, if the radiography operator operates the portable terminal with the panel held in his one-side arm and the portable terminal held in the other hand when quick and secure operations are required when the photography is carried out at a bedside.

In accordance with the second aspect of the present invention, a medical image photographing system comprises:
an information management apparatus for readably holding photographing order information having a patient ID particular to a patient to be photographed medical images and radiographing conditions having radiographic part, and for sending the photographing order information outside according to an inputted read command;
a control apparatus for obtaining the photographing order information from the information management apparatus through a communication network built in a hospital; and
a portable terminal for displaying the photographing order information obtained from the control apparatus on a screen, obtaining the information relating to the particular patient out of the displayed photographing order information, obtaining a panel identification information particular to a radiographing panel to be used for photographing a medical image of the patient, and storing into a memory the obtained panel identification information made correspondence with the photographing order information,
wherein the portable terminal has an input unit capable of being held and inputting with one hand for inputting various commands, and inputs, with input operations by the input unit, a command to display on the screen the photographing order information obtained from the control apparatus, a command to obtain the information relating to the particular patient out of the displayed photographing order information, a command to obtain the panel identification information particular to the radiographing panel to be used for photographing the medical image of the patient, and a command to store into the memory the obtained panel identification information made correspondence with the photographing order information.

According to this invention, since a radiography operator can easily operate the portable terminal with one hand only, the radiography operator can operate the portable terminal quickly and securely even if the radiography operator holds both the radiographing panel and the portable terminal, that is, if the radiography operator operates the portable terminal with the panel held in his one-side arm and the portable terminal held in the other hand when quick and secure operations are required when the photography is carried out at a bedside.

Preferably, the portable terminal displays the photographing order information on the screen so as to scroll the display according to a command inputted by the input unit capable of being held and inputting with one hand.

Preferably, the portable terminal exchanges the screen displayed on the screen according to a command inputted by the input unit capable of being held and inputting with one hand.

Preferably, the portable terminal deletes the obtained photographing order information from the memory according to a command inputted by the input unit capable of being held and inputting with one hand.

Preferably, the portable terminal further comprises an input unit for reading the panel identification information from the radiographing panel.

Preferably, the portable terminal sends to the control apparatus the photographing order information made correspondence with the panel identification information, and erases from the memory the photographing order information already sent to the control apparatus.

Preferably, the portable terminal further has a strap capable of being hung with the terminal held.

According to this invention, because the radiography operator can hang the portable terminal on his neck, the radiography operator can avoid unexpected accident such as suddenly dropping the portable terminal when he is operating the portable terminal with one hand.

Preferably, the portable terminal further comprises other operational input unit than the input unit capable of being held and inputting with one hand, and the operational input unit can be set to invalid inputting.

According to this invention, if the portable terminal has other operational input unit than the input unit capable of being held and inputting with one hand, because this operational input unit can be set to invalid inputting, an unexpected error input can be prevented.

In accordance with the third aspect of the present invention, a medical image photographing system comprises:
an information management apparatus for readably holding photographing order information having a patient ID particular to a patient to be photographed medical images and radiographing conditions having radiographic part, and for sending the photographing order information outside according to an inputted command;
a control apparatus for obtaining the photographing order information from the information management apparatus through a communication network built in a hospital; and
a portable terminal for obtaining the photographing order information from the control apparatus and displaying the photographing order information on a screen;
wherein the portable terminal displays on the screen a screen having an input screen for selectively inputting a particular patient ID out of patient IDs comprised in the photographing order information, and a screen having an input screen for inputting a panel identification information particular to a radiographing panel for use in a medical photography with respect to the patient ID which is selectively inputted, selectively inputs the patient ID and inputs the panel identification information through each two input screens.

According to this invention, the photographing order information displayed on the screen of the portable terminal is a very minimum of (for example, an essential information of) prompt and reliable photography at the bedside of a hospital room, which allows curtailing the time took in the preparation for the photography when the medical photographing is conducted at the bedside, and avoiding a wrong operation which can occur in the preparation for the photography.

Preferably, the portable terminal can transit the screen between two screens.

Preferably, the portable terminal displays as a reference screen the input screen for selectively inputting the particular patients ID out of the patient IDs.

Preferably, the portable terminal scrollably displays two screens in a longitudinal direction and/or a lateral direction.

### BRIEF DESCRIPTION OF THE DRAWINGS

The present invention will become more fully understood from the detailed description given hereinbelow and the accompanying drawings which are given by way of illustration only, and thus are not intended as a definition of the limits of the present invention, and wherein;
FIG. 1 is a schematic block diagram showing the configuration of a medical image photographing system in accordance with the present invention;
FIG. 2 shows a state that a radiography operator carries a radiographing panel C and a portable terminal 4 shown in FIG. 1;
FIG. 3 shows a state that the radiography operator holds the portable terminal 4 shown in FIG. 1 in his palm;
FIG. 4 is a block diagram showing the internal configuration of an information management apparatus shown in FIG. 1;
FIG. 5 is a block diagram showing the internal configuration of the portable terminal shown in FIG. 1;
FIG. 6 is a block diagram showing the internal configuration of a control apparatus shown in FIG. 1;
FIG. 7 is a flowchart explaining the information processing executed by the information management apparatus shown in FIG. 1;
FIG. 8 is a flowchart explaining the communication processing executed by the portable terminal shown in FIG. 1;
FIG. 9 is a flowchart explaining the communication processing executed by the control apparatus shown in FIG. 1;
FIG. 10 is a flowchart explaining the image processing executed by the control apparatus shown in FIG. 1;
FIG. 11 shows an example of the contents of the photographing order information;
FIG. 12A shows an example that patient names and IDs among photographing order information are displayed on the screen of the portable terminal shown in FIG. 1; and FIG. 12B shows an example that radiographing conditions and panel identification information for an individual patient among photographing order information are displayed on the screen of the portable terminal shown in FIG. 1;
FIG. 13 shows an example in which hospital room, age, and sex are displayed out of the photographing order information on the screen of the portable terminal shown in FIG. 1;
FIG. 14A shows an example, for the case of unfinished and normal photography, of screens for instruction to display the photographing order information on the screen of the control apparatus shown in FIG. 1; and FIG. 14B shows an example, for the case of unfinished and portable photography, of screens for instruction to display the photographing order information on the screen of the control apparatus shown in FIG. 1;
FIG. 15 is one example of an input screen for inputting patient information displayed on the screen of the control apparatus shown in FIG. 1;
FIG. 16 is one example of an input screen for inputting the radiographing conditions displayed on the screen of the control apparatus shown in FIG. 1;
FIGS. 17A, 17B and 17C are one example of the list of the photographing order information displayed on the screen of the control apparatus shown in FIG. 1; and
FIG. 18 is one example of radiographs displayed on the screen of the control apparatus shown in FIG. 1.

### DETAILED DESCRIPTION OF THE PREFERRED ENBODIMENTS

The present invention will be hereinafter described in details

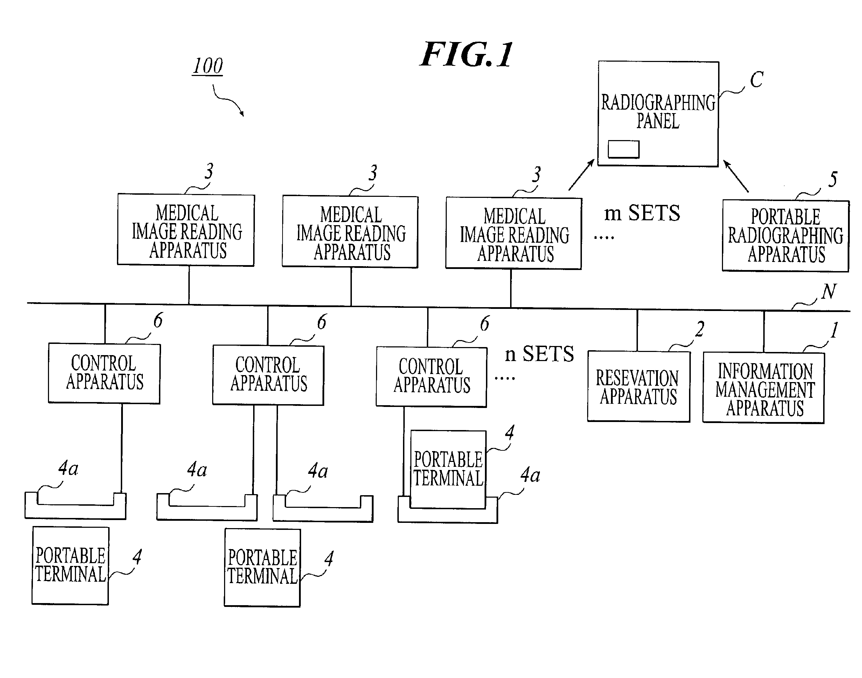

Referring first to FIG. 1, which is a schematic block diagram showing the configuration of a medical image photographing system 100, the configuration of the medical image photographing system 100 will be outlined.

As shown in FIG. 1, the medical image photographing system 100 is installed in hospital facilities and has an information management apparatus 1, a reservation apparatus 2, medical image reading apparatus 3, portable terminals 4, communication terminals 4a, a portable radiographing apparatus 5, and control apparatus 6.

Further, the information management apparatus 1, the reservation apparatus 2, the medical image reading apparatus 3, and the control apparatus 6 can mutually transmit and receive data through a network N, such as a LAN (Local Area Network) or a WAN (Wide Area Network), built in the hospital facilities.

Further, one set of control apparatus 6 is wired with one or more sets of communication terminals 4a in advance for transmitting and receiving data to and from the portable terminal 4. The installed number of the medical image reading apparatus 3 and the control apparatus 6 is flexible, and, for example, m sets of medical image reading apparatus 3 and n sets of control apparatus 6 are supposed to be installed. Each control apparatus 6 can communicate with any one of m sets of medical image reading apparatus 3 through the network N, and can obtain radiograph data from any medical image reading apparatus 3.

The information management apparatus 1, the reservation apparatus 2 and the portable radiographing apparatus 5 can also be installed by plural sets in the hospital, especially, the portable radiographing apparatus 5 may be arranged at a given space or a nurses' station (both not shown) located on each floor of the hospital.

The information management apparatus 1, when receiving photographing order information sent from the HIS/RIS or the control apparatus 6, stores the received photographing order information.

The photographing order information has an additional flag (hereinafter "portable indicating flag") indicating either of two types of photographing order information; one is that for carrying out radiography at the radiographing room (hereinafter referred to as "normal photography"), and the other one is that for carrying out radiography at the bedside of a hospital room using the portable radiographing apparatus 5 (hereinafter "portable radiography"). ON of the portable indicating flag means the photographing order information for the portable radiography, and OFF means that for the radiography of the radiographing room.

When the information management apparatus 1 receives the photographing order information with panel identification information added sent from the control apparatus 6, the information management apparatus 1 updates the photographing order information from the previously stored one to that with panel identification information added. The information management apparatus 1 also stores the radiograph data obtained from the medical image reading apparatus 3.

Here, the configuration and operation of the information management apparatus 1 and the photographing order information having the additional panel identification information will be explained later in detail.

The reservation apparatus 2 are installed at an examination reception desk and/or an examination room, and each apparatus 2 is an input terminal into which a doctor or the like inputs the photographing order information on the radiography. PC terminals, which are installed at the examination reception desk and the examination room, correspond to the reservation apparatus 2. The reservation apparatus 2 sends the inputted photographing order information to the HIS/RIS.

The medical image reading apparatus 3 reads the radiograph data from the radiographing panel C set on a reading part (not shown), where the radiographing panel C is an imaging plate in which a photostimulable phosphor layer is formed, or a cassette incorporating this imaging plate (both, not shown). The medical image reading apparatus 3 causes the imaging plate to emit fluorescence from the photostimulable phosphor layer corresponding to the accumulated radiation energy, converts the emitted fluorescence photoelectrically into radiograph data and outputs them.

The radiographing panel C, namely, the imaging plate and/or the cassette has its own bar code particular to the radiographing panel C as a panel identification information affixed on its surface. The panel identification information is not particularly limited to this way, as long as it can identify the radiographing panel C. For example, an IC chip having a panel identification information recorded therein may be employed.

The portable terminal 4 is a portable information terminal that the radiography operator, who operates the portable radiographing apparatus 5, carries with him when the photography is carried out. The portable terminal 4 is prepared at a predetermined place, for example, a nurses' station within the hospital accommodating the patient, together with the communication terminal 4a, the radiographing panel C and the portable radiographing apparatus 5.

The portable terminal 4 is set on the communication terminal 4a, and communicates with the control apparatus 6, which is wired with the communication terminal 4a, when data transmission and reception between the control apparatus 6 becomes ready. The portable terminal 4 transmits and receives data to and from only particular control apparatus 6 that is wired with the communication terminal 4a.

The portable terminal 4 is, for example, a PDA (Personal Digital Assistant), or may be a note-type PC. Further, the communication terminal 4a may be a cradle, for example.

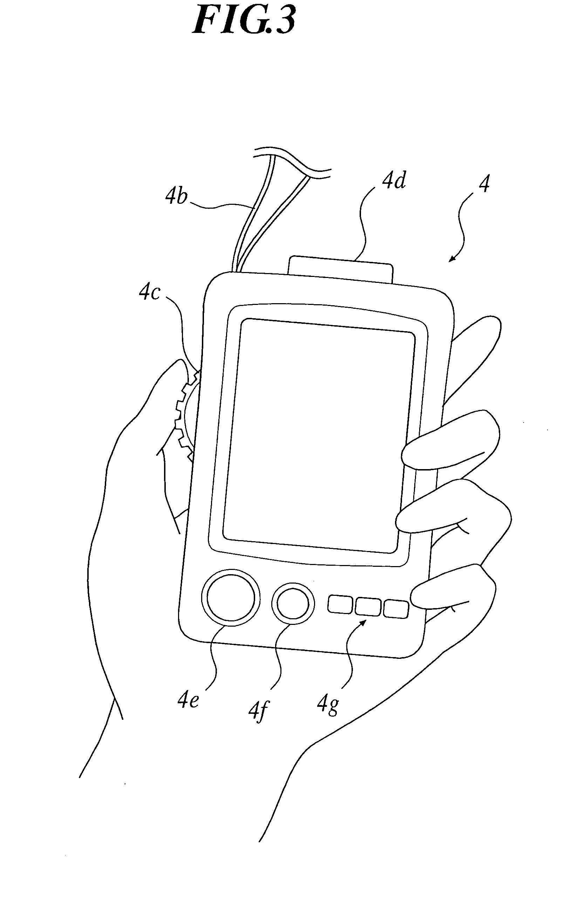

Further, the portable terminal 4 has a strap 4b as shown in FIG. 3, and if the radiography operator hangs the portable terminal 4 on his neck with the strap 4b, the radiography operator can avoid falling down the portable terminal 4 even when the portable terminal 4 slips from his hand. Therefore, the radiography operator does not need to pay attention not to fall down the portable terminal 4, which allows the radiography operator safety working. Further, it is possible that the radiography operator holds the portable terminal 4 only at the time of operation, and otherwise works without holding the portable terminal 4 in his hands. The strap 4b may be such a type that is used being hung on his wrist.

In this case, the radiography operator can touch a jog dial 4c (an input unit capable of being held and inputting with one hand) on the portable terminal 4 with the thumb, as shown in FIG. 3, and handle the jog dial 4c with the thumb only to operate the portable terminal 4 itself. Accordingly, the portable terminal 4 can be operated very easily. The portable terminal 4 also has plural operational input units 4e to 4g other than the jog dial 4c.

The portable terminal 4 also has a bar code reader 4d removably mounted thereon for reading bar code information recorded on the surface of the cassette and/or the radiographing panel C. The configuration and operation of the portable terminal 4 will be described in detail later.

The portable radiographing apparatus 5 is a radiographing apparatus movable between hospital rooms and beds for carrying out radiography (X-ray) with use of a cassette at the bedside of an individual room.

The control apparatus 6 transmits and receives data to and from the portable terminal 4 through one or more sets of wired communication terminals 4a, that is, can communicate with a particular portable terminal 4 only through the communication terminal 4a.

Also, the control apparatus 6 downloads photographing order information from the information management apparatus 1, and sends the downloaded photographing order information to each portable terminal 4 through the wired communication terminal 4a. The control apparatus 6 can also input and edit the photographing order information, and upload to the information management apparatus 1 the photographing order information after inputting and editing or the photographing order information having the additional panel identification information sent from the portable terminal 4.

The control apparatus 6 is also capable of displaying the radiograph data obtained from the medical image reading apparatus 3, whereby the radiographic operator can confirm the photographed result easily. In this case, it is preferable to install both of the medical image reading apparatus 3 and the control apparatus 6 at a same room or a same floor. This installation helps the radiography operator confirm the photographed result without moving room to room or floor to floor.

The configuration and operation of the control apparatus 6 will be described later in detail.

In other words, when the photographing order information, which is inputted from the HIS/RIS or the control apparatus 6, is sent to the portable terminal 4, the radiography operator takes up the portable terminal 4, refers to the photographing order information displayed on the screen of the portable terminal 4, and confirms the patient to be carried out radiography at the bedside and the necessary size and the sheet number of the radiographing panels C. Then, the radiography operator carries the radiographing panels C of the confirmed size and the sheet number, the portable terminal 4 and the portable radiographing apparatus 5 to the corresponding bedside of the hospital room accommodating the patient to be photographed. Every time the radiography operator photographs, he records in the portable terminal 4 the correspondence between the photographing order information and the radiographing panel C (namely, panel identification information), and sets the portable terminal 4 on the communication terminal 4a after photography. At this time, the photographing order information with the panel identification information added is automatically uploaded from the portable terminal 4 to the information management apparatus 1.

The portable terminal 4, collating the patient ID inputted by the radiography operator with that comprised in the photographing order information, displays the collated result on the screen of a display part 43, or may have a function of audible notification. The patient ID may be preferably inputted into the portable terminal 4 using a bar code, which improves reliability because the work to confirm the patient is performed by a computer not by the eye of the radiography operator.

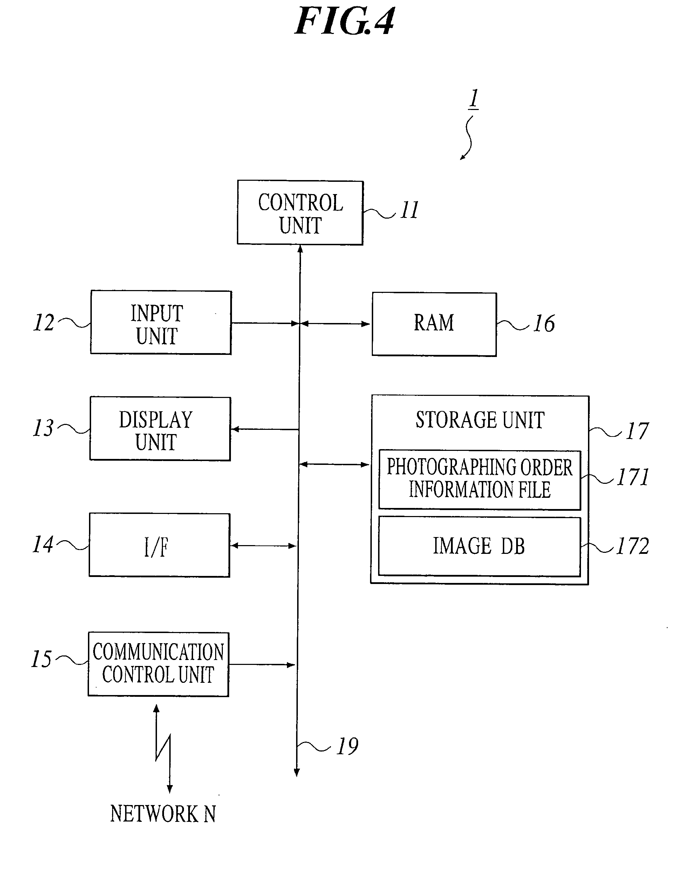

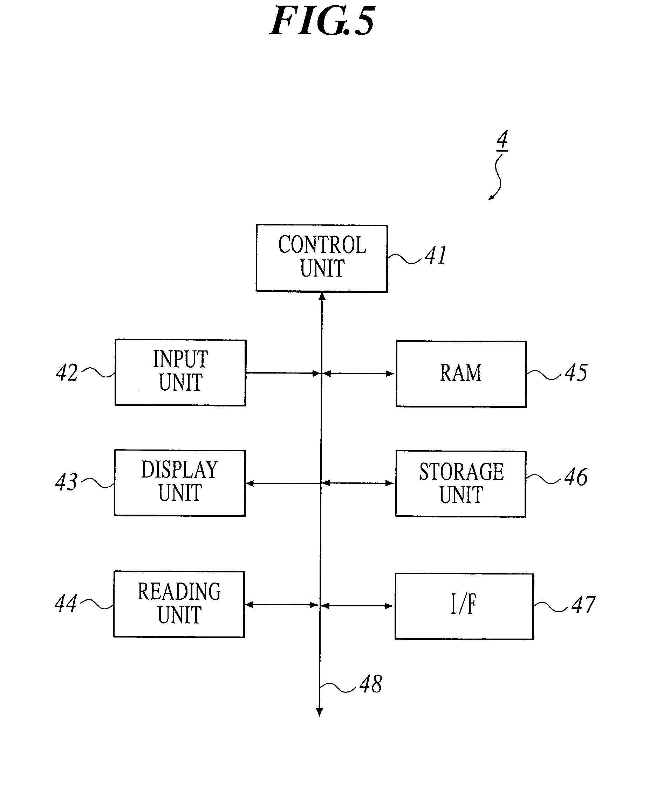

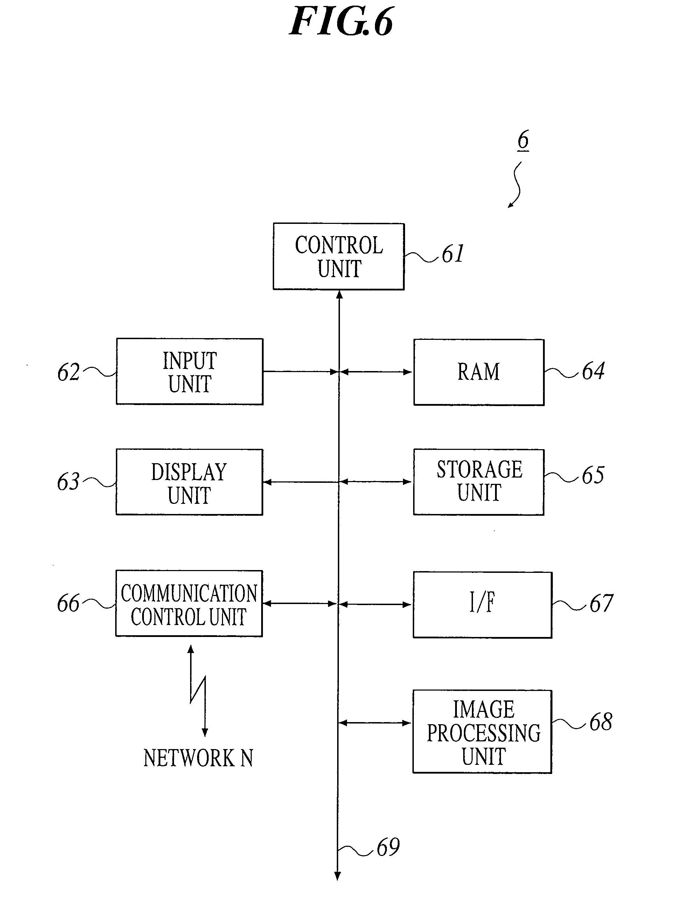

Next, the configuration of the information management apparatus 1, the portable terminal 4 and the control apparatus 6 will now be explained in detail with reference to FIGS. 4 to 6. FIG. 4 shows the internal configuration of the information management apparatus 1, FIG. 5 that of the portable terminal 4, and FIG. 6 that of the control apparatus 6.

First, the configuration of the information management apparatus 1 will be explained.

As shown in FIG. 4, the information management apparatus 1 has a control unit 11, input unit 12, display unit 13, I/F 14, communication control unit 15, RAM 16, storage unit 17, and image processing unit 18, and these units are connected to each other through a bus 19.

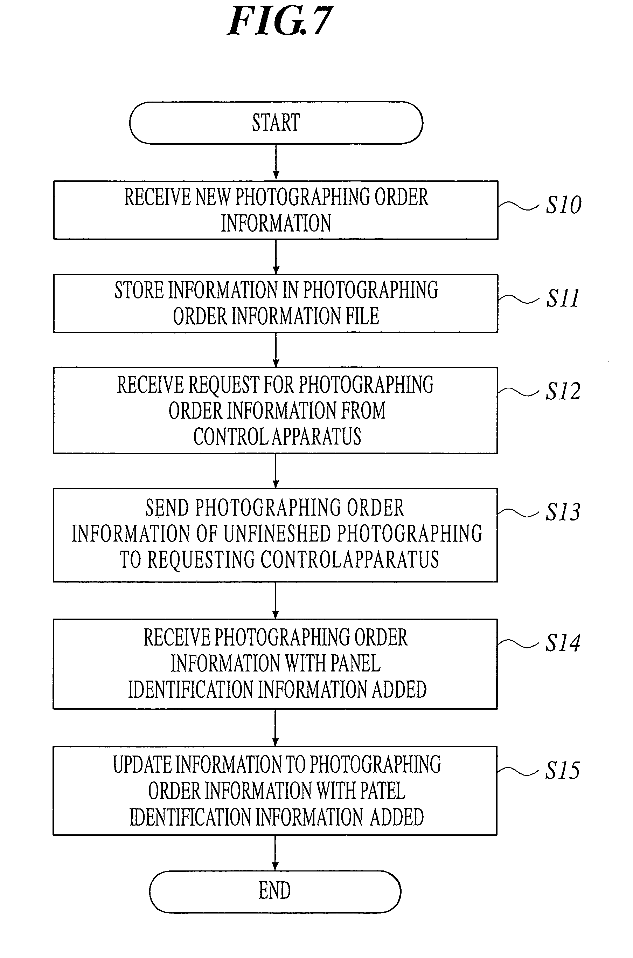

The control unit 11 reads out and executes various programs stored in the storage unit 17, the program being especially for information processing shown in a flow chart of FIG. 7, which will be explained later in detail.

The control unit 11, receiving the photographing order information sent from the HIS/RIS or the control apparatus 6, records the photographing order information into a photographing order information file 171. When receiving the photographing order information having the additional panel identification information sent from the control apparatus 6, the control unit 11 updates the photographing order information previously recorded in the photographing order information file 171 to that having the panel identification information. The control unit 11 also stores the radiograph data obtained from the medical image reading apparatus 3 into an image DB (data base) 172.

The input unit 12 has a keyboard having cursor keys, numeric input keys, various function keys, etc. and a pointing device such as a mouse (both, not shown), and outputs to the control unit 11 command signals inputted with key operations of the keyboard and mouse operations. Further, the input unit 12 may have a touch panel mounted on the screen of the display unit 13, and output to the control unit 11 the command signals inputted through the touch panel.

The display unit 13 has a not-shown screen, such as an LCD (Liquid Crystal Display) and a CRT (Cathode Ray Tube), and displays various display data according to display control signals from the control unit 11.

The I/F 14 is an interface that not only transmits and receives data to and from the control apparatus 6 but also receives the photographing order information from the HIS or RIS.

The communication control unit 15 has a LAN board, a router or a terminal adapter (TA) (not shown), and controls communication between each apparatus connected to the network N.

The RAM (Random Access Memory) 16 extends to executable form various programs that are read from the storage unit 17 by the control unit 11 and stores those various programs. Further, the RAM 16 temporarily stores various data produced during program execution by the control unit 11.

The storage unit 17 has, for example, an HDD (Hard Disc Drive), writable/erasable nonvolatile semiconductor memory or the like, and stores various programs and various data. The storage unit 17 especially stores the program for execution of the information processing shown in the flow chart of FIG. 7, which will be explained later in detail.

The storage unit 17 has the radiographic order information file 171, and the image DB 172. The radiographic order information file 171 records the photographing order information inputted from the HIS/RIS or the control apparatus 6, and the image DB 172 stores the radiograph data applied image processing by the control apparatus 6, by corresponding the patient ID information thereto.

The storage unit 17 may have a recording medium (not shown) having previously stored various programs and various data. This recording medium is a nonvolatile memory, such as a magnetic recording medium, an optical recording medium, or a semiconductor memory, to be mounted fixedly or removably to the storage unit 17.

Next, the configuration of the portable terminal 4 will be explained. The portable terminal 4 incorporates a chargeable battery (not shown) that supplies electric power to the portable terminal 4 for working itself.

As shown in FIG. 5, the portable terminal 4 has a control unit 41, input unit 42, display unit 43, reading unit 44, RAM 45, storage unit 46 and I/F 47, and these units are connected to each other through a bus 48.

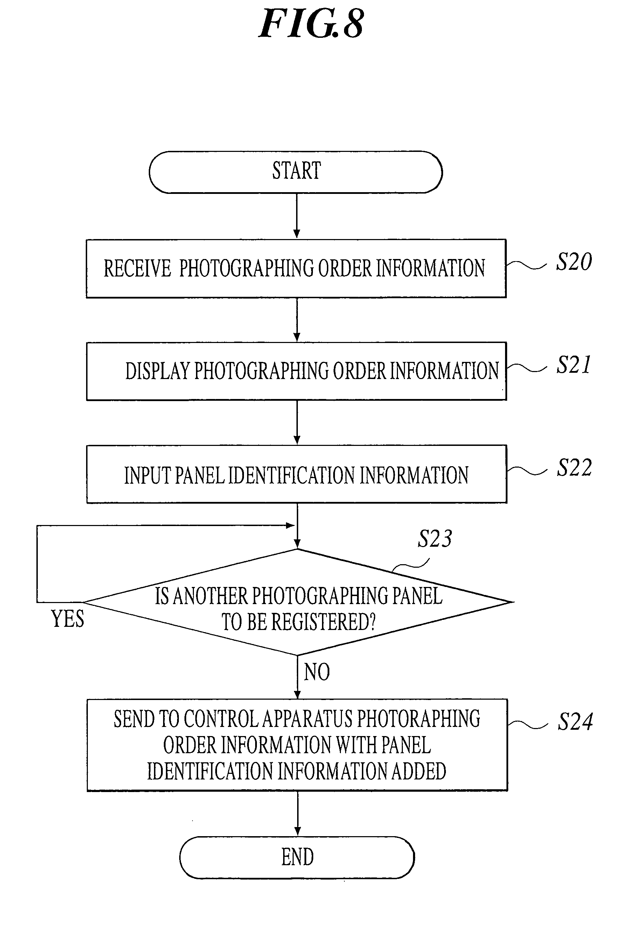

The control unit 41 has a CPU (not shown), and reads out and executes various programs stored in the storage unit 46. The control unit 41 especially reads out the program from the storage unit 48 and executes it. The program is to execute information processing shown in a flow chart of FIG. 8, which will be explained later in detail.

It is preferable that the control unit 41 controls the input unit 42, which will be explained later, so as to enable the jog dial 4c only to be used when the portable terminal 4 is set to a radiographing mode. This control helps the radiography operator to avoid an unexpected operation such as an erroneous key input when carrying out radiography.

When the portable terminal 4 is set on the communication terminal 4a, the control unit 41 communicates data with the control apparatus 6 previously wired with the communication terminal 4a.

When the portable terminal 4 is set on the communication terminal 4a after registration of the panel identification information, the control unit 41 sends to the control apparatus 6 the photographing order information with the panel identification information added through the communication terminal 4a, and erases the data already sent.

If the radiography operator holds the portable terminal 4, that is, if the radiography operator once held the portable terminal 4 and sets it again on the communication terminal 4a without data communication with the control apparatus 6, the control unit 41 rejects the registration of the same panel identification information.

The control unit 41 has a function of sorting the photographing order information received from the control apparatus 6, based on the items comprised in the photographing order information. These items are, for example, the name of a patient, patient ID information, sex, age, hospital room (having a ward and a floor), patient location (the location of a bed), requesting department, requesting doctor's name, regions to be photographed, the size and the sheet number of radiographing panels, operator ID of the radiography operator in charge, a flag showing complete/incomplete photography, etc. The control unit 41 also has a function of sorting under arbitrary combination of plural items of the photographing order information.

Further, the control unit 41 has a function of displaying on the screen of the display unit 43 the photographing order information such as a number and/or a size of cassette necessary for the order of radiographing patient and for its photography.

The input unit 42 has cursor keys, numeric input keys, and various function keys such as plural operational members 4e to 4g, and outputs to the control unit 41 command signals inputted by the key operations of these various keys.

The input unit 42 has the jog dial 4c. The dial operation of the jog dial 4c outputs to the control unit 41 command signals for scrolling (moving) various displayed parts on the screen of the display unit 43, and an input (determine) command for the scrolled displayed part is outputted to the control unit 41 by pressing down the jog dial 4c.

Optionally, the input unit 42 may have a touch panel mounted on the screen of the display unit 43, and output to the control unit 41 the command signals inputted through the touch panel.

The display unit 43 has a screen such as an LCD (not shown) to display various display data according to display control signals from the control unit 41, and particularly displays the photographing order information obtained from the control apparatus 6 through the communication terminal 4a.

The display unit 43 in this embodiment has one screen per one surface of the portable terminal 4 as shown in FIG. 3, but, without being limited to this case, may have one screen per each of two surfaces consisting of the front and rear of the portable terminal 4. In the latter case, the information that goes over the display capacity of one screen can be displayed on the other screen, thereby conveniently saving additional operation of the jog dial 4c for moving the screen.

The reading unit 44 has a removable bar code reader 4d, and outputs to the control unit 41 the bar code information such as the panel identification information read by the bar code reader 4d.

The RAM 45 extends to executable form various programs that are read from the storage unit 46 by the control unit 41 and stores those various programs. Further, the RAM 45 temporarily stores various data produced during program execution by the control unit 41, the data being, for example, the photographing order information, the panel identification information read through the reader unit 44, and the like.

The storage unit 46 has an HDD, nonvolatile semiconductor memory or the like, and stores various programs and various data. The storage unit 46 especially stores the program for execution of the communication processing shown in the flow chart of FIG. 8, which will be explained later in detail.

The storage unit 46 may have a recording medium (not shown) having stored various programs and various data. This recording medium is a nonvolatile memory, such as a magnetic recording medium, an optical recording medium, or a semiconductor memory, to be mounted fixedly or removably to the storage unit 46.

The I/F 47 is an interface for communicating data with the control apparatus 6 through the communication terminal 4a, the interface conforming to RS-232C, IrDA (Infrared Data Association) or the like.

Here, the communication terminal 4a is wired with only one control apparatus 6, and has a communication control unit (not shown), for controlling data communication with the portable terminal 4 set into a slot (not shown).

Both of the portable terminal 4 and the communication terminal 4a have respective non-contact communication terminals, through which data are transmitted and received. Accordingly, even if the portable terminal 4 is frequently set to the communication terminal 4a, the non-contact communication terminals are free of abrasion, thereby improving reliability.

Although the portable terminal preferably employs a jog dial, a touch-key system can be used.

Further, the portable terminal 4 utilizes products available in the general market. In this case, it is preferable that the operational input members 4e to 4g other than the jog dial 4c are made not usable. Such a function can be easily realized by installing corresponding software into the portable terminal 4.

Further, the portable terminal 4 incorporates a removable and chargeable battery (not shown), which is charged while the portable terminal 4 is placed on the communication terminal 4a.

In order to protect data from being rewritten by accidentally touching the jog dial 4c while the portable terminal 4 is held, it is preferable that the portable terminal 4 has a locking function so as to prohibit data modification after photography, (that is, after inputting given data). Such a function can be easily realized by installing corresponding software into the portable terminal 4.

Further, when the portable terminal 4 is taken out from the communication terminal 4a and used at a bedside, the portable terminal 4 can use only the functions of inputting, displaying and storing data, and cannot transmit or receive data to and from other equipments such as the control apparatus 6. With this feature, the portable terminal 4 can avoid affecting other medical equipments used at the bedside, such as heart pacemakers and the like, without influence of radio waves emitted from the portable terminal 4.

Next, the configuration of the control apparatus 6 will be explained.

Referring to FIG. 6, the control apparatus 6 has a control unit 61, input unit 62, display unit 63, communication control unit 66, RAM 64, storage unit 65, I/F 67 and image processing unit 68, and these units are connected to each other through a bus 69.

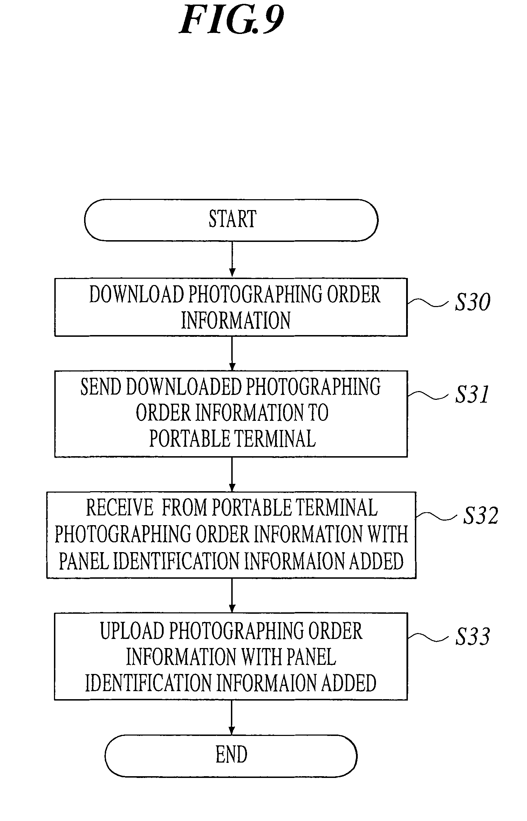

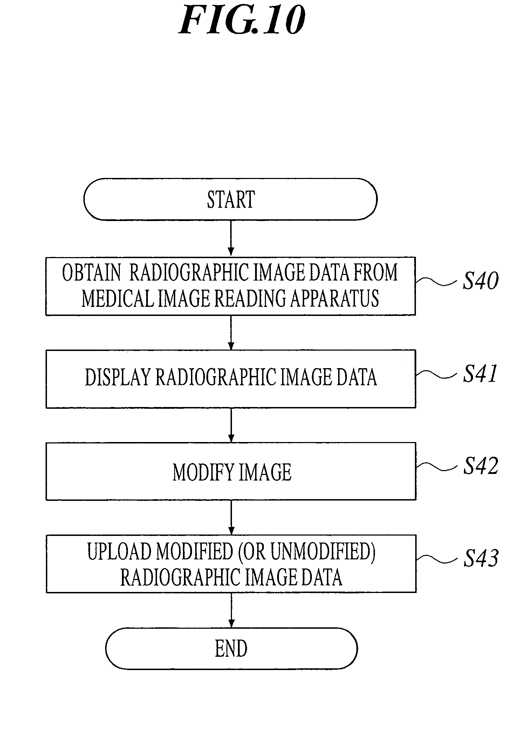

The control unit 61 reads out and executes various programs stored in the storage unit 65. The control unit 61 especially reads out the program from the storage unit 65 and executes it. The program is to execute communication processing and image processing shown in the flow chart of FIGS. 9 and 10, respectively, which will be explained in detail later.

The control unit 61 obtains radiograph data from the medical image reading apparatus 3, selects a process pattern (having frequency processing, gradation processing, rotation processing, enlarge/shrink processing) corresponding to radiographing conditions, such as radiographic parts, indicated in the photographing order information pertaining to the obtained radiograph data, and commands the image processing unit 68 to execute image processing (having compression/expansion processing) based on the process pattern.

The control unit 61 stores into the image DB 172 the radiograph data processed by the image processing unit 68, by corresponding the photographing order information thereto. Meanwhile, the process pattern information corresponding to the radiographing conditions is stored in the storage unit 65 in advance.

The input unit 62 has a keyboard having cursor keys, numeric input keys, various function keys, etc. and a pointing device such as a mouse (both, not shown), and outputs to the control unit 61 command signals inputted with key operations of the keyboard and mouse operations. Further, the input unit 62 may have a touch panel mounted on the screen of the display unit 63, and output to the control unit 61 the command signals inputted through the touch panel.

The display unit 63 has a screen, such as an LCD and a CRT (not shown), and displays various display data according to display control signals from the control unit 61.

The RAM 64 extends to executable form various programs read from the storage unit 65 by the control unit 61 and stores those various programs. Further, the RAM 64 temporarily stores various data produced during program execution by the control unit 61.

The storage unit 65 has an HDD, nonvolatile semiconductor memory or the like, and stores various programs and various data. The storage unit 65 especially stores the program for execution of the communication processing and the image processing shown in FIGS. 9 and 10, respectively, which will be explained later in detail. The storage unit 65 also stores plural process patterns for image processing corresponding to the radiographing conditions.

The storage unit 65 may have a recording medium (not shown) having previously stored various programs and various data. This recording medium is a nonvolatile memory, such as a magnetic recording medium, an optical recording medium, or a semiconductor memory, to be mounted fixedly or removably to the storage unit 65.

The communication control unit 66 has a LAN board, a router or TA (not shown), and controls communication between apparatus connected to the network N.

The I/F.67 is an interface for communicating data with the portable terminal 4 through the communication terminal 4a, the interface conforming to RS-232C, IrDA or the like.

With respect to the radiograph data obtained from the medical image reading apparatus 3 under the control of the control unit 61, the image processing unit 68 selects the process pattern corresponding to the radiographing conditions indicated in the photographing order information pertaining to the obtained radiograph data, and executes image processing (having the compression/expansion processing) based on the process pattern.

Next, a description will now be given of operations of the information management apparatus 1, the portable terminal 4 and the control apparatus 6 with reference to FIGS. 7 to 10.

FIG. 7 is a flowchart explaining the information processing executed by the information management apparatus 1, FIG. 8 is a flowchart explaining the communication processing executed by the portable terminal 4, and FIG. 9 and FIG. 10 are flowcharts explaining the communication processing and the image processing, respectively, executed by the control apparatus 6.

Referring first to FIG. 7, the operation relating to the information processing of the information management apparatus 1 will be explained.

When the control unit 11 receives a new photographing order information from HIS/RIS or the control apparatus 6 (step S10), the unit 11 stores the new photographing order information into the photographing order information file 171 (step S11).

When receiving a signal requesting the photographing order information from the control apparatus 6, the control unit 11 selects unfinished photographing order information out of the photographing order information stored in the file 171, and sends it to the requesting control apparatus 6 (step S13). Here, the unfinished photographing order information means the photographing order information to which a panel identification information has not yet been added.

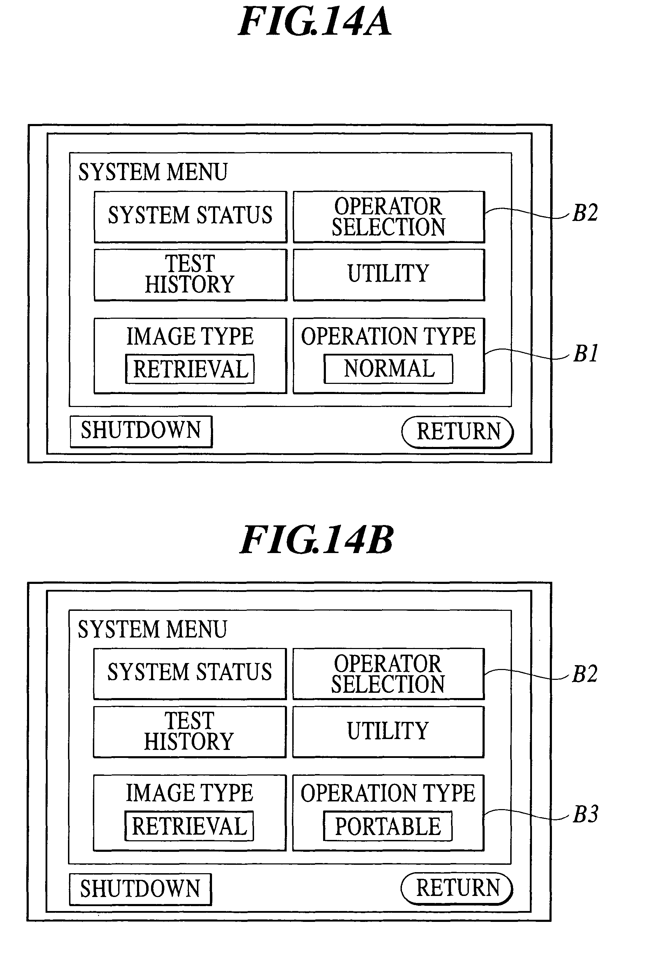

If a plurality of control apparatus 6 is enabled, and all control apparatus 6 request the photographing order information, the unfinished photographing order information is sent to all control apparatus 6. The photographing order information is requested by selectively inputting a display position B1 shown in FIG. 14A or a display position B3 shown in FIG. 14B.

At step S13, when the control apparatus 6 requests the control unit 11 the photographing order information, particularly for portable radiography, the control unit 11 refers to a portable indicating flag previously added to the photographing order information out of the photographing order information stored in the photographing order information file 171, selects the unfinished photographing order information for portable radiography and sends it to the requesting control apparatus 6.

When receiving from the control apparatus 6 the photographing order information with the panel identification information added (step S14), the control unit 11 updates the corresponding photographing order information to the received photographing order information with the panel identification information added (step S15). Also, when the unit 11 receives photographing order information to be updated from the control apparatus 6, the corresponding photographing order information is updated to the photographing order information to be updated.

Next, referring to FIG. 8, the communication processing by the portable terminal 4 will be explained.

The control unit 41 receives photographing order information from the control apparatus 6 when the portable terminal 4 is set on the communication terminal 4a (step S20), and displays the received photographing order information on the screen of the display unit 43 (step S21). That is, the photographing order information is automatically loaded in the portable terminal 4 installed in the ward accommodating the patient, which allows the radiography operator to promptly prepare photographing images within the ward.

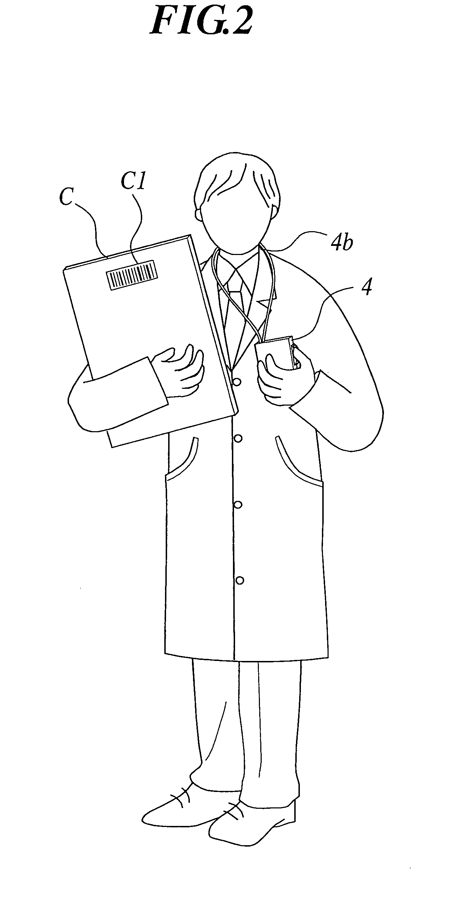

When the radiography operator holds the portable terminal 4 (see FIG. 2 and FIG. 3) after step S21 and inputs some commands through the input unit 42, the control unit 41 functions according to the inputted commands.

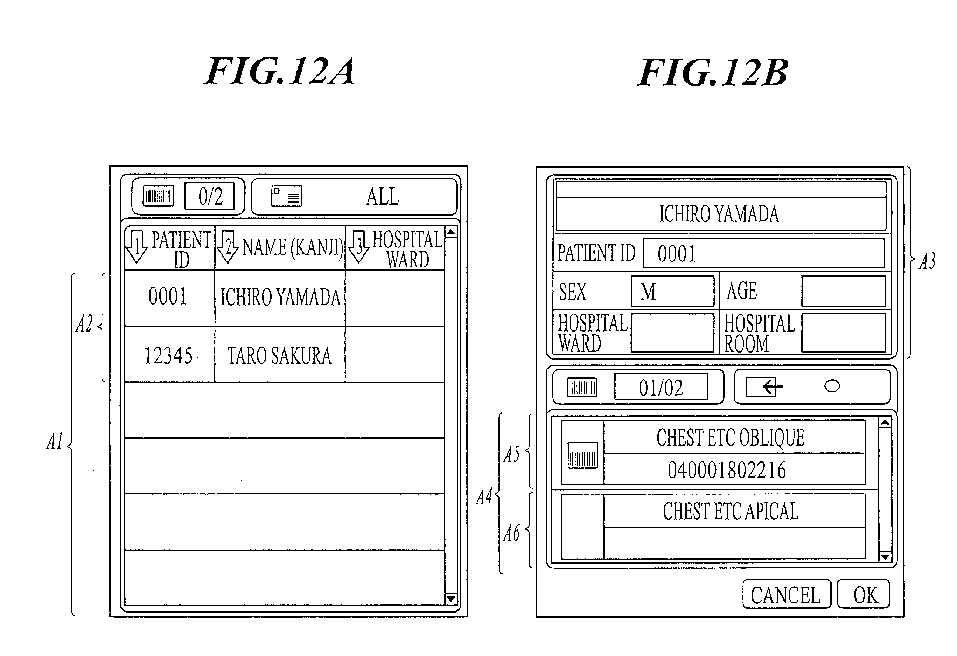

For instance, in a hospital room, when the radiography operator confirms the matching between the patient information (patient name, patient ID) of the photographing order information displayed on the screen 43 and that for an actual patient to be photographed, and inputs the command of reading the panel identification information on the radiographing panel C to be photographed for each radiographing condition indicated at display position A4, the control unit 41 reads the panel identification information (bar code information) on each radiographing panel C directly from the corresponding radiographing panel through the bar code reader 4d of the reading unit 44 (step S22, and S23; Yes). Specifically, at display position A5 of FIG. 12B, there are presented "CHEST ETC OBLIQUE" as an radiographic part out of the radiographing conditions, as well as a panel identification information; 04000108022016 of the radiographing panel for photographing the corresponding radiographic part read at step S23. These steps are repeated according to the number of photography, and made one-to-one correspondence between the radiographing condition and the radiographing panel.

In this case, the radiography operator, holding the radiographing panel C to be used for the photography in his one-side arm, brings the.bar code reader 4d on the portable terminal 4 held in his other hand close to the panel identification information indicating place, and presses down the jog dial 4c at a readable area to read the panel identification information of the radiographing panel C.

Further, it is preferable not to read the panel identification information at one time for all radiographing panels to be photographed, but to read the panel identification information for every photographing operation. This reading method prevents the radiographing panel from being used for other photographing with different radiographing condition from the corresponding condition to be applied.

If the control unit 41 reads all panel identification information, that is, finishes photographing and registration of radiographing panels (step S23; No), the portable terminal 4 is set on the communication terminal 4a and, when communication with the control apparatus 6 is ready, sends to the control apparatus 6 the photographing order information to which the panel identification information added corresponding to the finished photography, and also erases the data already sent out (step S24).

In the above process, FIG. 12A and FIG. 12B are display examples of the photographing order information, which are displayed on the screen of the display unit 43. FIG. 12A is a display example showing patient names and patient IDs, and FIG. 12B is a display example showing radiographing conditions directed for each patient.

The photographing order information displayed on the screen of the portable terminal 4 must be a very minimum of (for example, an essential information of) prompt and reliable photography at the bedside of a hospital room. In view of this, the present inventor or the like found that (1) the operation for selecting the radiograph order or the patient from the list of the photographing order information and (2) the operation for registering the radiographing panel to be used for the photography, or for associating the radiograph order and the radiographing panel C are necessary for the radiography operator to carry out radiograph by using the portable terminal 4 at the bedside of a hospital room. The portable terminal 4 has a function for automatically displaying each two screens respectively corresponding to the essential two operations on the screen of the portable terminal 4. This two-screen displaying will be hereinafter described in detail.

The screen shown in FIG. 12A is a screen for the operation shown in the above described (1), that is, the operation for selecting the radiograph order or the patient from the list of the photographing order information. On the screen shown in FIG. 12A, patient names and patient IDs are displayed in a list form at display position A1, specifically at display position A2, there are displayed a patient name "ICHIRO YAMADA" and its patient ID "0001", and a patient name "TARO SAKURA" and its patient ID "12345", for two patients. The radiography operator operates the jog dial to select the patient name and the patient ID of one patient out of these two patients (thereby, the operation shown in the above described (1) is finished), and then, under the condition the patient name and the patient ID are selected, presses down the jog dial to display the radiographing conditions associated with the selected patient. For example, the radiography operator selects the patient name "ICHIRO YAMADA" and its patient ID "0001" shown at the display position A2 of FIG. 12A by operating the jog dial, and displays the radiographing conditions associated with the patient of the patient name "ICHIRO YAMADA" and its patient ID "0001" as shown in FIG. 12A by pressing down the jog dial. Here, the screen shown in FIG. 12B is a screen for the operation shown in the above described (2), that is, the operation for registering the radiographing panel to be used for the photography, or for associating the radiograph order and the radiographing panel C.

On the screen shown in FIG. 12B, there are displayed in a list form a patient name "ICHIRO YAMADA" and its patient ID "0001", etc. at display position A3, and radiographing conditions "CHEST ETC OBLIQUE" and its panel identification information "04000108022016" (see display position A5), and another radiographing condition "CHEST ETC APICAL" (see display position A6), etc. in display position A4. The dial operation of the jog dial enables the display position A4 to be scrolled, which allows displaying radiographing conditions over the screen capacity of the display position A4. Further, the operation of the above described (2) is respectively finished when the panel identification information is added to each radiographing condition shown in the display position A4.

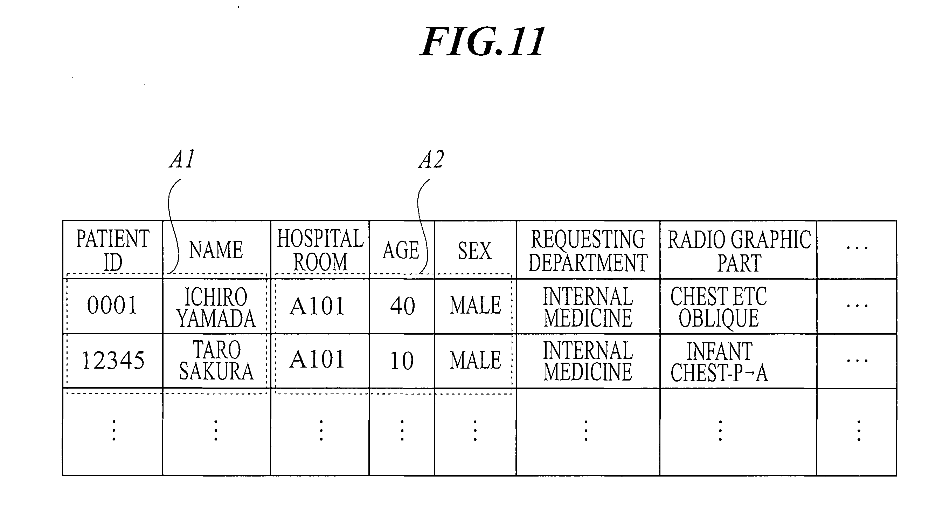

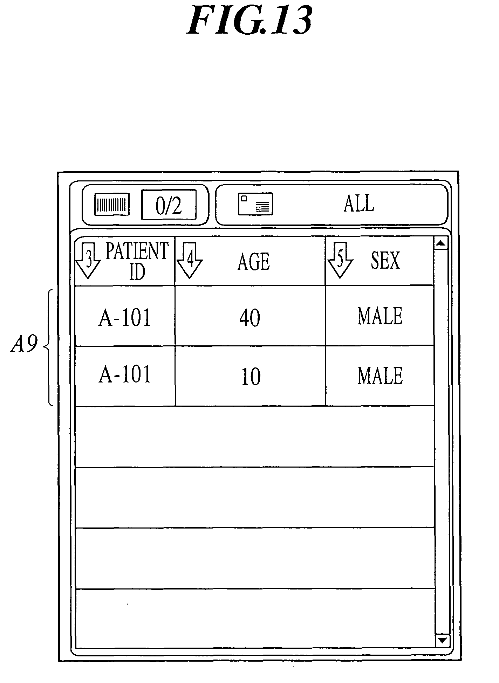

Further, although the contents displayed on the screen shown in FIG. 12A are the contents shown in the figure code A1 out of the photographing order information shown in FIG. 11 that the portable terminal 4 receives from the control device 6, the other contents of the photographing order information shown in FIG. 11 are displayed on the screen of the portable terminal 4 when the screen shown in this FIG. 12A is scrolled. For example, the contents shown in the figure code A2 is displayed at the display position A9 of the screen shown in FIG. 13, out of the contents of the photographing order information shown in FIG. 11.

Therefore, on the screen of the portable terminal 4, although any contents can be displayed out of the contents of the photographing order information shown in FIG. 11, in case the information other than the contents of the photographing order information is displayed on the screen of the display unit 43 (for example, in case the screen shown in FIG. 12B is displayed), the control unit 41 is sure to display on the screen of the display unit 43 the screen shown in FIG. 12A or the contents shown in the figure code A1 out of the contents of the photographing order information when the screen is changed (except scrolled) to the screen displaying the photographing order information in accordance with the command inputted through the input unit 42. That is, the control unit 41 displays on the screen of the display unit 43 the screen shown in FIGS 12A and 12B when the screen is respectively changed (except scrolled).

Further, if one reference screen is transited to the next screen by scrolling it, it transits to the reference screen shown in FIGS 12A and 12B.

Next, the operation of the control apparatus 6 will be explained.

The operation associated with the communication processing of the control apparatus 6 will be first explained referring to FIG. 9.

The control unit 61 displays on the screen of the display unit 63 the screen shown in FIG. 14A or FIG. 14B according to the direction of the radiography operator, when the photographing order information is downloaded from the information management apparatus 1 or newly inputted. At this time, if there is inputted through the input unit 62 a command of obtaining all photographing order information for unfinished and normal photography, the screen of FIG. 14A is displayed on the screen of the display unit 63, and if commanded to obtain all photographing order information for unfinished and portable radiography, the screen of FIG. 14B is displayed on the screen of the display unit 63.

When an input button "OPERATOR SELECTION" shown at display position B2 on the screen of FIGS. 14A or 14B is keyed in through the mouse or the touch panel of the input unit 62, listed radiography operator information is sent from the information management apparatus 1, which allows the selection of a radiography operator to be engaged from the list.

When an input button "OPERATION TYPE (NORMAL)" shown at display position B1 in FIG. 14A is keyed in through the mouse or the touch panel of the input unit 62, the control unit 61 downloads all photographing order information for unfinished and normal photography from the information management apparatus 1, and when "OPERATION TYPE (PORTABLE)" shown at display position B2 in FIG. 14B is keyed in through the mouse or the touch panel, the control unit 61 downloads all photographing order information for unfinished and portable photography from the information management apparatus 1 (step S30).

In this case, if the photographing order information for unfinished and portable photography is not registered in the information management apparatus 1, the radiography operator inputs new photographing order information for portable radiography, or, keying in the input button "OPERATION TYPE (NORMAL)" shown at the display position B1, downloads all photographing order information for unfinished and normal photography from the information management apparatus 1 and, if necessary, changes any one of these photographing order information for normal photography to that for portable radiography. In other words, when the photographing order information for normal photography is downloaded, the radiography operator chooses photographing order information for portable radiography to be set for portable radiography out of the downloaded photographing order information for normal photography, and sets to ON the portable indicating flag of the chosen photographing order information.

Referring back to FIG. 9, the control unit 61 sends the order information for portable radiography to the portable terminal 4 set in the communication terminal 4a, through the communication terminal 4a connected to its own control apparatus 6 (step S31). If the photographing order information for portable radiography is newly inputted one or that changed from normal photography, the control unit 61 uploads the photographing order information to the information management apparatus 1, too.

When the control unit 61 receives the photographing order information with the panel identification information added, that is, receives the photographing order information of finished photography from the portable terminal 4 set in the communication terminal 4a, through the communication terminal 4a connected to its own apparatus 6 (step S32), the control unit 61 uploads the received photographing order information with the panel identification information added to the information management apparatus 1 (step S33).

Next, the operation associated with the image processing of the control apparatus 6 will be explained with reference to FIG. 10.

The control unit 61 obtains radiograph data, which relates to the photographing order information communicated with the portable terminal 4, from the medical image reading apparatus 3 (step S40). There are generally arranged m sets of medical image reading apparatus 3, and the control unit 61 sends a request for obtaining the radiograph data to all m sets of medical image reading apparatus 3.

When the data is requested, the medical image reading apparatus 3 determines if a memory (not shown) stores the radiograph data associated with the panel identification information corresponding to each panel identification information sent from the control apparatus 6. If the radiograph data having a matched panel identification information is stored in the memory, the radiograph data are sent to the requesting control apparatus 6.

After image processing by the image processing unit 68, the control unit 61 displays the radiograph data obtained at step S40 together with the photographing order information on the screen of the display unit 63 (step S41) .

After step S41, according to a command to modify an image entered through the input unit 62, the control unit 61 modifies the image for the radiograph data (step S42), and uploads the modified radiograph data together with the photographing order information to the information management apparatus 1 (step S43).

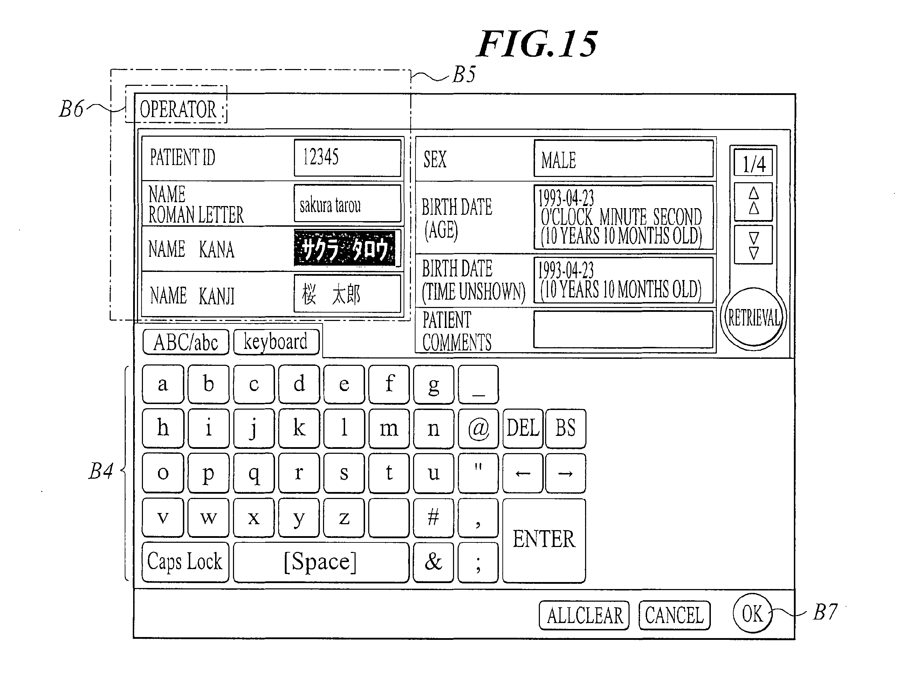

Next, a description will now be given of an input operation of the new photographing order information in the control apparatus 6 with reference to FIGS. 15 to 17 (refer to steps S31 and S32 shown in FIG. 9). In the following description, it is assumed that the new photographing order information is that for portable radiography. The display control is executed to the screens of the display unit 63 shown in FIGS. 15 to 17 are controlled by the control unit 61.

Initially, when the registration of new photographing order information is commanded, a screen, for example, shown in FIG. 15 is displayed on the screen of the display unit 63, and patient information, such as a patient name and a patient ID, relating to the new photographing order information are inputted through the input unit 62.

Here, input keys are displayed at display position B4 on the screen, and key input operation to the input key displayed on the screen is carried out through the mouse or the touch panel of the input unit 62. The key input operation may be carried out through the keyboard of the input unit 62. At display position B5, there are displayed input portions for the patient ID and the name (roman letter, kana, and kanji), respectively. Displayed at display position B6 is the operator name that is selected when the input button "OPERATOR SELECTION" shown at display position B2 on the screen of FIGS. 14A or 14B is keyed in through the mouse or the touch panel.

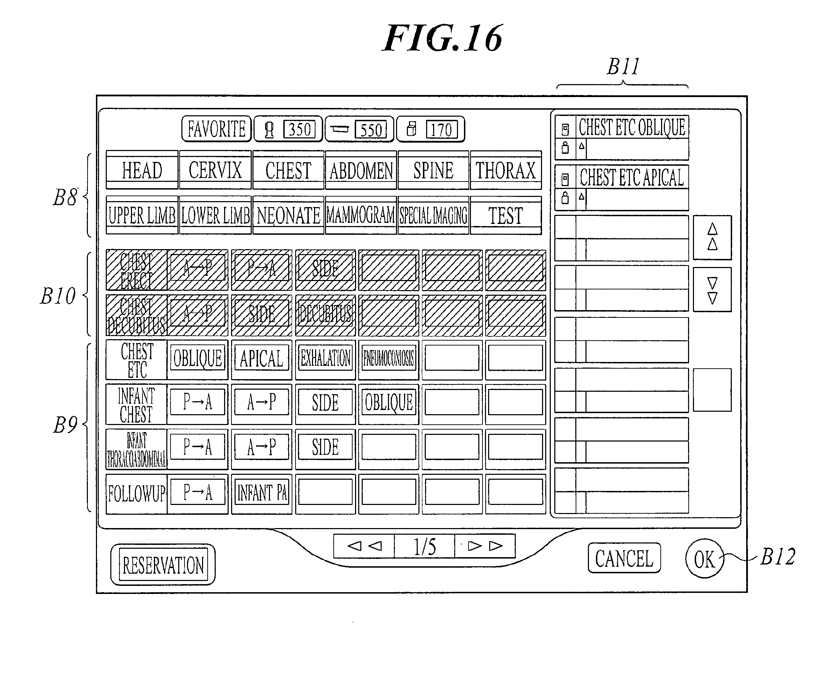

When display position B7 is keyed in through the mouse or the touch panel, a screen, for example, shown in FIG. 16 is displayed on the screen of the display unit 63, which allows the setting of radiographing conditions corresponding to the patient name and the patient ID displayed at the input unit of display position B5. Displayed at display position B8 are selection buttons for main classification items of the radiographing conditions, such as "HEAD, NECK, ---, TEST". When any one of the classification items of the main classification items is keyed in, there are displayed, at display positions B9 and B10, more detailed classification items of the radiographing conditions corresponding to the keyed-in item (for example, CHEST), such as "CHEST ERECT (A→P,--, SIDE),----, PROGRESS OBSERVATION (P→A, INFANT PA)". When being keyed in, for example, "CHEST ETC OBLIQUE" and "CHEST ETC APICAL" out of the classification items at display position B9, the keyed-in radiographing conditions are displayed at display position B11 in a list form.

Here, radiographing conditions displayed with halftone dot meshing at display position B10 are that impossible to be photographed as portable radiography. Thus, the items of radiographing conditions can be displayed in different displaying forms according as the portable radiography is possible or impossible.

In the embodiment explained above, the items of radiographing conditions for the possible or impossible portable radiography are displayed in different displaying ways, but it is not limited to this method. For instance, with the items of radiographing conditions displayed in the same displaying ways for the possible or impossible portable radiography, when an item of radiographing condition for impossible portable radiography is erroneously keyed in, refusal of the key input may be notified by an audible or visible warning.

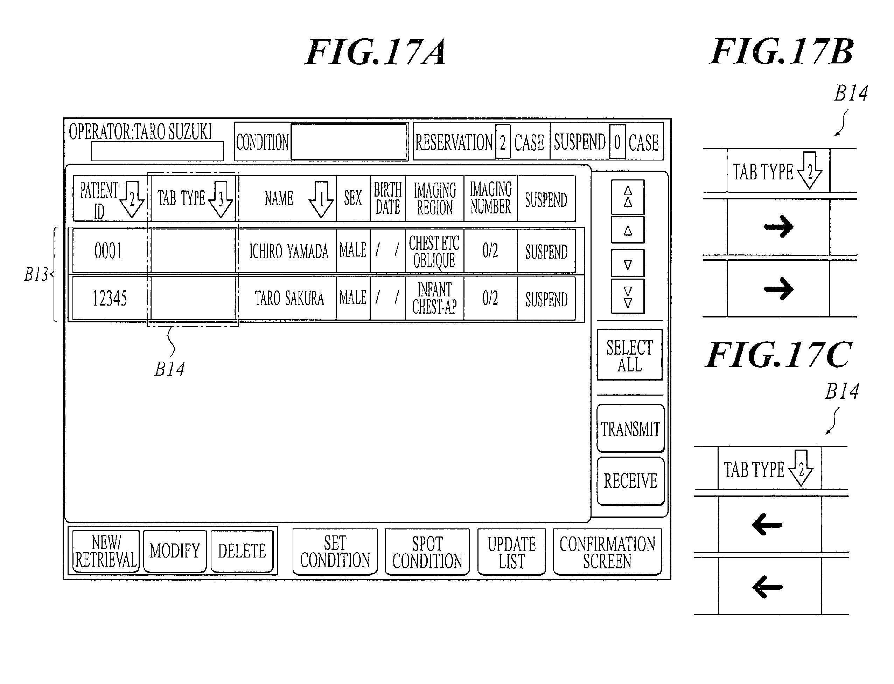

When display position B12 is keyed in through the mouse or the touch panel, a screen, for example, as shown in FIG. 17A is displayed on the screen of the display unit 63, displaying in a list the patient name, the patient ID, etc. with radiographing conditions input, corresponding to the radiographing conditions. Specifically, displayed in a list at display position B13 are registered two patients, namely, a patient having a patient ID "0001" and a patient name "ICHIRO YAMADA", and another patient having the patient ID "12345" and the patient name "TARO SAKURA".

Here, when the photographing order information for respective patients as listed at display position B13 are sent from the control apparatus 6 to the portable terminal 4, symbols "→" (rightward arrow) are displayed for indicating the sending of the information as shown, for example, at display position B14 in FIG. 17B. When the photographing order information sent to the portable terminal 4 is sent back from the portable terminal 4 with a panel identification information added (that is, as the photographing order information for finished photography), symbols "←" (leftward arrow) are displayed as shown at display position B14 in FIG. 17C. As an alternative, other symbols may be used instead of symbols "→" and "←".

If an input button "CANCEL" displayed on the screen shown in FIGS. 15 or 16 is keyed in, the new photographing order information in the midst of the entry is canceled by the control unit 61. Even after completing the entry of the new photographing order information, if an input button "DELETE" displayed on the screen shown in FIGS. 17A, 17B and 17C is keyed in, the completed photographing order information is canceled by the control unit 61.

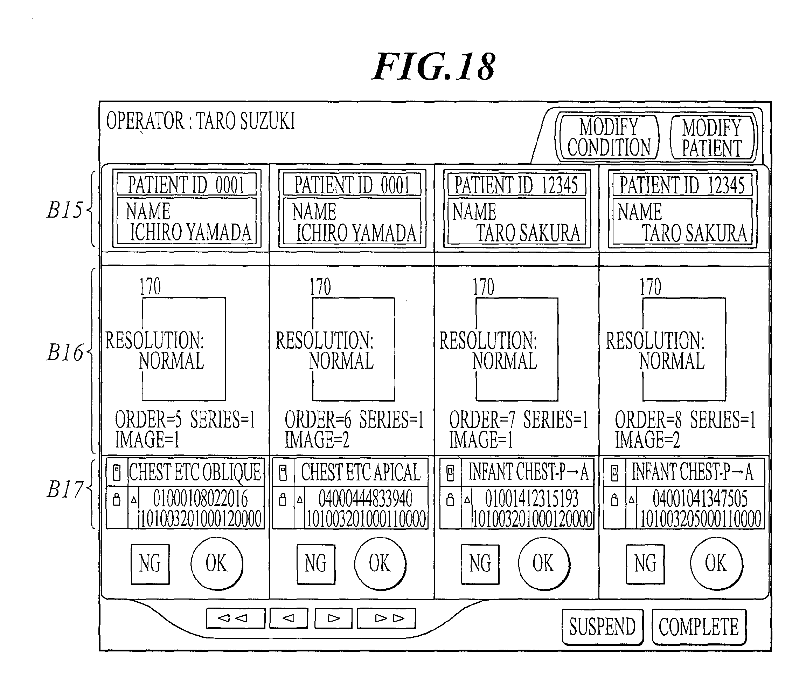

Next, a description will be given of radiographs displayed on the screen of the display unit 63 of the control apparatus 6 with reference to FIG. 18 (refer to steps S41 and S42 shown in FIG. 10). The radiograph data in the following description are those photographed by the portable radiography. The display control is executed to the screen of the display unit 63 shown in FIG. 18 by the control unit 61.

When the radiograph data radiographed by the portable radiography are obtained from the medical image reading apparatus 3, the data are displayed, for example, on the screen of the display unit 63 shown in FIG. 18. That is, the radiograph data obtained from the medical image reading apparatus 3 are those processed by each portable terminal 4, and the radiographs relating to respective plural radiographing conditions for plural patients, which is processed by one portable terminal 4, are displayed in a parallel manner. Specifically, for example at display positions B15 to B17, there are displayed in a parallel manner two radiographs for patient name "ICHIRO YAMADA" and its patient ID "0001", and two radiographs for patient name "TARO SAKURA" and its ID "12345". If the radiographs cannot be displayed on one screen because of the capacity, scrolling the screen allows displaying the radiographs.

Here, patient IDs and patient names are displayed at display position B15, radiographs at display position B16, and radiographing conditions, its identification numbers, and panel identification information used for the photography at display position B17.

As described above, the medical image photographing system 100 has the information management apparatus 1 for managing the photographing order information and the radiograph data, the medical image reading apparatus 3 for reading the radiograph data from the radiographing panel C, the portable terminal 4 for making correspondence between the photographing order information and the panel identification information of the radiographing panel corresponding to each radiographing condition, the portable radiographing apparatus 5 capable of carrying out radiography (X-ray) at a bedside of a hospital room, and the control apparatus 6 for obtaining the photographing order information from the information management apparatus 1 and inputting the new photographing order information, as well as for obtaining and displaying the radiograph data from the medical image reading apparatus 3.

The portable terminal 4 has the jog dial 4c easily operable with one hand, and the strap 4b to be hung on the neck of the radiography operator holding the portable terminal 4.

Further, the photographing order information which is a very minimum of prompt and reliable photography at the bedside of a hospital room and which is like the screen shown in FIGS 12A and,12B is preferentially displayed on the screen of the portable terminal 4.

With this arrangement, the radiography operator can operate the portable terminal 4 with his one finger (for example, thumb) only, holding the portable terminal 4 in his hand, and can easily operate the communication terminal 4a. In case of the portable radiographing at the bedside, the radiography operator holds the radiographing panel and the portable terminal simultaneously, and has to operate the portable terminal 4 with one hand. Even in such a case, the radiography operator can easily operate the portable terminal 4. This structure is especially convenient when the panel identification information on the radiographing panel C, which is held in one arm, is read by the bar-code reader 4d on the portable terminal 4 held in the other hand.

Further, since the portable terminal 4 has the strap 4b, if the radiography operator hangs the portable terminal 4 on his neck with the strap 4b, the radiography operator can avoid falling down the portable terminal 4 even when the portable terminal 4 slips from his hand. Therefore, the radiography operator does not need to pay attention not to fall down the portable terminal 4, which allows the radiography operator safety working. Further, it is possible that the radiography operator holds the portable terminal 4 only at the time of operation, and otherwise works without holding the portable terminal 4 in his hands.

Therefore, the photographing order information displayed on the screen of the portable terminal 4 is a very minimum of (for example, an essential information of) prompt and reliable photography at the bedside of a hospital room, which allows curtailing the time took in the preparation for the photography when the medical photography is conducted at the bedside, and avoiding a wrong operation which can occur in the preparation for the photography.

For instance, although the communication terminal 4a, namely the portable terminal 4 in the embodiment communicates data only with the particular control apparatus 6 wired therewith, the communication terminal 4a, namely the portable terminal 4, instead, may communicate data with any control apparatus 6 comprised in the medical image photographing system 100.

Further, although the portable terminal 4 may be a jog dial type allowing the one-handed operation, it may also be a touch key type whereby the radiographic operator can operate it by touching direct to the screen.

Further, the portable terminal 4 may hold the jog dial and the touch-key input thereof (that is, not allow inputting thereon) so as not to wrongly rewrite the data when the photography is finished at the bedside and the potable terminal 4 is backed to the communication terminal 4a, and the held portable terminal 4 is released by backing it to the communication terminal 4a.

The entire disclosure of Japanese Patent Application No. Tokugan-2003-091535 and Japanese Patent Application No. Tokugan-2003-092407 filed on March 28, 2003 is incorporated herein by reference in its entirety.

## Claims

1. A medical image photographing system comprising:
an information management apparatus for readably holding photographing order information having a patient ID particular to a patient to which a photography of medical images is carried out and radiographing conditions having radiographic part, and for sending the photographing order information outside according to an inputted command;
a control apparatus for obtaining the photographing order information from the information management apparatus through a communication network; and
a portable terminal for displaying the photographing order information obtained from the control apparatus on a screen, obtaining the information relating to the particular patient out of the displayed photographing order information, obtaining a panel identification information particular to a radiographing panel to be used for photographing a medical image of the patient, and storing into a memory the obtained panel identification information made correspondence with the photographing order information,
wherein the portable terminal has input keys for inputting various commands, and inputs, with input operations by the input keys, a command to display on the screen the photographing order information obtained from the control apparatus, a command to obtain the information relating to the particular patient out of the displayed photographing order information, a command to obtain the panel identification information particular to the radiographing panel to be used for photographing the medical image of the patient, and a command to store into the memory the obtained panel identification information made correspondence with the photographing order information.

2. A medical image photographing system comprising:
an information management apparatus for readably holding photographing order information having a patient ID particular to a patient to be photographed medical images and radiographing conditions having radiographic part, and for sending the photographing order information outside according to an inputted read command;
a control apparatus for obtaining the photographing order information from the information management apparatus through a communication network; and
a portable terminal for displaying the photographing order information obtained from the control apparatus on a screen, obtaining the information relating to the particular patient out of the displayed photographing order information, obtaining a panel identification information particular to a radiographing panel to be used for photographing a medical image of the patient, and storing into a memory the obtained panel identification information made correspondence with the photographing order information,
wherein the portable terminal has an input unit capable of being held and inputting with one hand for inputting various commands, and inputs, with input operations by the input unit, a command to display on the screen the photographing order information obtained from the control apparatus, a command to obtain the information relating to the particular patient out of the displayed photographing order information, a command to obtain the panel identification information particular to the radiographing panel to be used for photographing the medical image of the patient, and a command to store into the memory the obtained panel identification information made correspondence with the photographing order information.

3. The system of claim 2, wherein the portable terminal displays the photographing order information on the screen so as to scroll the display according to a command inputted by the input unit capable of being held and inputting with one hand.

4. The system of claim 2 or 3, wherein the portable terminal exchanges the content displayed on the screen according to a command inputted by the input unit capable of being held and inputting with one hand.

5. The system of one of claims 2 to 4, wherein the portable terminal deletes the obtained photographing order information from the memory according to a command inputted by the input unit capable of being held and inputting with one hand.

6. The system of one of claims 1 to 5, wherein the portable terminal further comprises an input unit for reading the panel identification information from the radiographing panel.

7. The system of one of claims 1 to 6, wherein the portable terminal sends to the control apparatus the photographing order information made correspondence with the panel identification information, and erases from the memory the photographing order information already sent to the control apparatus.

8. The system of one of claims 2 to 7, wherein the portable terminal further has a strap capable of being hung with the terminal held.

9. The system of one of claims 2 to 8, wherein the portable terminal further comprises other operational input member than the input unit capable of being held and inputting with one hand, and the operational input member can be set to invalid inputting.

10. A medical image photographing system comprising:
an information management apparatus for readably holding photographing order information having a patient ID particular to a patient to be photographed medical images and radiographing conditions having radiographic part, and for sending the photographing order information outside according to an inputted command;
a control apparatus for obtaining the photographing order information from the information management apparatus through a communication network; and
a portable terminal for obtaining the photographing order information from the control apparatus and displaying the photographing order information on a screen;
wherein the portable terminal displays a content having an input screen for selectively inputting a particular patient ID out of patient IDs comprised in the photographing order information, and a content having an input screen for inputting a panel identification information particular to a radiographing panel for use in the medical photography with respect to the patient ID which is inputted and selected, selectively inputs the patient ID and inputs the panel identification information through each two input screens.

11. The system of claim 10, wherein the portable terminal can transit the screen between two screens.

12. The system of claim 10 or 11, wherein the portable terminal displays as a reference screen the input screen for selectively inputting the particular patient ID out of the patient IDs.

13. The system of one of claims 10 to 12, wherein the portable terminal scrollably displays two screens in a longitudinal direction and/or a lateral direction.
